(19) Europäisches Patentamt / European Patent Office / Office européen des brevets

(11) **EP 3 784 295 B1**

(12) **FASCICULE DE BREVET EUROPEEN**

(45) Date de publication et mention de la délivrance du brevet:
**17.04.2024   Bulletin 2024/16**

(21) Numéro de dépôt: **19719296.6**

(22) Date de dépôt: **24.04.2019**

(51) Classification Internationale des Brevets (IPC):
**A61L 2/00** *(2006.01)*    **A61Q 19/00** *(2006.01)*
**A61K 8/06** *(2006.01)*    **A61L 2/04** *(2006.01)*

(52) Classification Coopérative des Brevets (CPC):
**A61L 2/0023; A61K 8/06; A61L 2/04; A61Q 19/00;**
A61K 2800/805; A61L 2202/21

(86) Numéro de dépôt international:
**PCT/EP2019/060548**

(87) Numéro de publication internationale:
**WO 2019/207018 (31.10.2019 Gazette 2019/44)**

(54) **PROCÉDÉ POUR LA FABRICATION D'UNE ÉMULSION DERMO-COSMÉTIQUE STÉRILE**

VERFAHREN ZUR HERSTELLUNG EINER STERILEN DERMOKOSMETISCHEN EMULSION

PROCESS FOR PRODUCTION OF A STERILE DERMOCOSMETIC EMULSION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **24.04.2018   FR 1870482**
**24.04.2018   FR 1870483**

(43) Date de publication de la demande:
**03.03.2021   Bulletin 2021/09**

(73) Titulaire: **Pierre Fabre Dermo-Cosmétique**
**81500 Lavaur (FR)**

(72) Inventeur: **DELAUNAY, Jean-Claude**
**34800 CLERMONT-L'HERAULT (FR)**

(74) Mandataire: **Ipside**
**Tour Trinity**
**1 B Place de la Défense**
**92400 Courbevoie (FR)**

(56) Documents cités:
**EP-A1- 1 588 697        WO-A2-99/32156**
**US-A1- 2017 354 160**

## Description

L'invention concerne un procédé pour la fabrication d'une émulsion dermo-cosmétique stérile. L'invention est plus particulièrement destinée à la stérilisation d'une préparation dermo-cosmétique sous la forme d'un gel, d'une crème ou d'un lait, comprenant une base émulsive, laquelle base émulsive est stérilisée par l'action de la chaleur.

## Domaine technique

Une telle préparation, lorsque le niveau de stérilité est suffisamment élevé et qu'elle est conditionnée dans un emballage adéquat, présente une longue durée de conservation, sans qu'il ne soit nécessaire d'y ajouter des produits conservateurs, tels que des esters de l'acide para-hydroxybenzoïque, des sorbates ou des esters de glycol. Ces adjuvants ne participent pas aux principes actifs visés pour les préparations qui les contiennent mais peuvent cependant être mal tolérés. Il est par conséquent souhaitable de proposer des produits, notamment dans le domaine dermo-cosmétique, n'utilisant pas de tels adjuvants conservateurs, mais qui présentent une durée de conservation et une propreté microbienne adéquates.

Ainsi, les formulations visées par le procédé objet de l'invention sont dépourvues de conservateurs, susceptibles d'être responsables d'intolérances cutanées, notamment de tout ammonium quaternaire, éthanol, phénols, amidines, dérivés d'isothiazolone, esters parahydroxybenzoïques (connus sous le nom de parabens), etc.

Le terme conservateur désigne ainsi toute substance apte à empêcher le développement de micro-organismes dans la composition par son action antimicrobienne propre.

Le terme conservateur, au sens de la présente invention, englobe aussi bien les conservateurs *stricto sensu,* notamment tels que réglementairement listés (par exemple dans le Règlement CE n° 1223/2009 du Parlement européen et du Conseil du 30 novembre 2009 relatif aux produits cosmétiques, définition article 2.1.I et Annexe V), que les substances dites « *preservative-like* », non listées par la réglementation, mais présentant tout de même une fonction de conservateur.

De telles substances sont susceptibles d'être présentes dans les formules cosmétiques pour des fonctions autres, telles que parfumer, tonifier, raffermir, etc., alors qu'elles présentent également des propriétés antimicrobiennes, qui empêchent ainsi la croissance des micro-organismes.

Les formulations des produits visés par le procédé objet de l'invention ne comprennent ni conservateurs listés dans l'Annexe V du Règlement CE n° 1223/2009 du Parlement Européen et du Conseil du 30 novembre 2009 relatif aux produits cosmétiques, ni conservateur non listé ou « *preservative-like* ». Ainsi, sont par exemple exclus de la formulation des produits visés par le procédé objet de l'invention, et compte tenu de leur effet de conservateur, les composés tels que le caprylyl glycol, le pentylèneglycol, l'éthylhexylglycérine, et plus généralement tout adjuvant à effet bactéricide, fongicide, bactériostatique ou fongistatique, notamment des parfums sur base alcoolique et des huiles essentielles présentant ces propriétés.

Ces produits dermo-cosmétiques stériles et sans aucune forme de conservateur, préservent le film hydrolipidique naturellement présent sur la peau lors de leur application.

La peau est en effet recouverte d'un film protecteur, appelé film hydrolipidique de surface. Ce film en constitue la barrière la plus externe, ainsi que la plus fragile, la plus perturbée et la plus représentative de la santé de la peau. Il est constitué en grande partie des corps gras excrétés par les glandes sébacées et des lipides provenant de la dégradation des cellules lors de la phase de kératinisation des cellules cornées, ainsi que de composés hydrophiles, tels que l'eau de la sueur, le glycérol, l'urée, les facteurs naturels d'hydratation de la peau, les sels, les métabolites de la flore cutanée, etc.

Ce film de surface est très exposé et très sensible aux stress environnementaux, aux habitudes d'hygiène, et à l'état de la peau. Il s'avère important de préserver, et même d'améliorer, cette fonction barrière, et ce d'autant plus pour les peaux les plus sensibles.

Ainsi, depuis leur introduction en 2012, les produits dermo-cosmétiques redevables du procédé objet de l'invention jouissent d'un fort succès commercial avec en moyenne la vente d'un tel produit toutes les 2 secondes dans le monde. En réponse à ce succès commercial, il est nécessaire de disposer de procédés de fabrication de ces produits en grande quantité sans en dégrader la qualité finale.

## Technique antérieure

Plusieurs méthodes sont utilisables pour obtenir une émulsion stérile, comme par exemple ceux décrits dans EP 1 588 697 A1 et WO 99/32156 A2.

Ces méthodes s'appliquent soit à la formulation dans son ensemble : la formulation est créée en mélangeant ses différents composants en les émulsionnant, puis la préparation est stérilisée, éventuellement dans son emballage ; ou, lesdites méthodes s'appliquent aux composants individuels de la formulation, qui sont préalablement stérilisés, puis mélangés sous environnement stérile et émulsionnés, pour obtenir la formulation.

La première méthode consistant à obtenir le produit avant sa stérilisation est plus productive.

Quelle que soit la méthode, la stérilisation est obtenue, par exemple, par un traitement thermique, consistant à porter le produit ou le composant à une température suffisante durant un temps suffisant pour tuer les micro-organismes contenus dans le produit, par un traitement mécanique tel qu'une ultrafiltration ou une exposition à des pressions extrêmes, ou encore par irradiation.

Dans un produit exempt de tout type de conservateur, tels que ceux exclus dans le cadre de l'invention, la conservation du produit est obtenue en stérilisant celui-ci à un haut degré de stérilité, comparable, voire supérieur, à celui utilisé pour des produits pharmaceutiques injectables.

La stérilité est définie par la norme EN 556 et la pharmacopée européenne en vigueur, comme la probabilité d'un micro-organisme de proliférer dans ledit produit. Typiquement, ladite probabilité, pour un produit stérile, est inférieure à $10^{-6}$.

Ce niveau de stérilité correspond à un indice de stérilité F0 égal à 15. Cette valeur F0, dont la méthode de détermination est définie par la pharmacopée européenne en vigueur, donne un temps, exprimé en minutes, quantifiant l'effet létal de la chaleur humide à 121°C sur les micro-organismes viables.

L'effet létal est mesuré par rapport à un germe de référence, le géobacillus stéarothermophilus sporulé, ces bactéries étant particulièrement résistantes et tolérantes à la chaleur.

Ainsi, l'effet de stérilisation minimum visé est celui qu'aurait produit sur ces germes, dans le milieu étudié, un traitement thermique de 15 minutes à 121°C. Ce traitement croit en temps de manière exponentielle avec la baisse de la température en fonction de la nature du micro-organisme dont la destruction est visée par le traitement. La valeur de F0 est ainsi donnée par la formule :

$$F0 = t.10^{(T-121/z)}$$

où :

- $t$ est le temps de traitement exprimé en minutes ;
- $z$ a la dimension d'une température et est défini par la résistance thermique du micro-organisme considéré. La valeur de $z$ est définie expérimentalement en regard d'un paramètre $D$.
- $D$ est un temps de réduction décimal et mesure le temps, à une température donnée, ici à 121°C, pour réduire la concentration du micro-organisme considéré de 90 %. Pour géobacillus stéarothermophilus, $D$ est égal à une minute.
- $T\ est$ la température du traitement, exprimée en °C dans cette formule.

Ainsi, $z$ est la variation de température qui modifie la valeur de $D$ d'un facteur 10, pour géobacillus stéarothermophilus, $z$ est égal à 10 °C, ces facteurs $D$ et $z$ sont fonction du milieu et varient notamment selon le type d'émulsion.

Ainsi la valeur de F0 est liée à la probabilité d'avoir un micro-organisme susceptible de proliférer dans le produit, ou plus précisément à l'abattement du nombre de micro-organismes contenus dans le produit avant et après l'opération de stérilisation.

Plus la température de traitement $T$ et le temps de maintien $t$ sont élevés et plus cette probabilité est faible, l'abattement étant d'autant plus élevé.

Ainsi, dans un produit non stérile comprenant $10^9$ germes par unité de volume, un traitement correspondant à un indice F0 de 15, produit un abattement de 15 Log du nombre de germes pour la même unité de volume, ramenant celui-ci à $10^{-6}$.

Ce niveau de stérilisation élevé permet une longue conservation du produit dès lors que celui-ci est conditionné dans un contenant évitant toute rétro-contamination, y compris pour des conditionnements de volume important, jusqu'à 400 ml.

Une stérilisation à 141°C et 10 secondes de maintien produit un F0 de 15, tout comme une stérilisation à 145°C et un temps de maintien de 4 secondes. Une stérilisation à 145°C pour un temps de maintien de 6 secondes produit un F0 de 22.

Lorsque la stérilisation n'est pas réalisée par l'action létale de la chaleur sur les micro-organismes, par exemple lorsque celle-ci est réalisée par ultrafiltration, par l'exposition à un faisceau d'électrons, à une lumière pulsée ou à une pression extrême, un F0 équivalent est déterminé par l'abattement du nombre de germes par unité de volume sous l'effet du procédé. Ainsi, tout procédé de stérilisation peut être caractérisée par une valeur F0 définissant l'abattement du nombre de micro-organismes qu'il réalise dans le produit traité.

**Problème technique**

Ainsi, un des problèmes techniques résolus par le procédé objet de l'invention est de pouvoir produire en grande quantité et avec une productivité élevée, des produits cosmétiques stériles avec un degré de stérilité élevé, typiquement pour un F0 supérieur ou égal à 15.

Les procédés de stérilisation, notamment par infusion, utilisés dans l'industrie agroalimentaire permettent des productivités élevées. Ces procédés sont des procédés thermiques qui soumettent le produit à une température très élevée (UHT) pendant un temps bref pour obtenir le niveau de stérilité visé.

Toutefois ces procédés sont généralement utilisés pour la mise en oeuvre de traitements atteignant un F0 de 3 à 7, nettement inférieur à ce qui est nécessaire pour les produits visés par l'invention.

Pour adapter ces procédés à la fabrication de produits dermo-cosmétiques stériles, il est nécessaire de chauffer plus et/ou de maintenir plus longtemps le produit à haute température. Dans ces conditions, le risque de dégrader le produit lors de ce traitement thermique augmente considérablement. Aussi, l'un des problèmes techniques visés par l'invention est de permettre la stérilisation d'un produit dermo-cosmétique à un haut degré de stérilité, par effet thermique, sans dégrader le produit.

Le document WO 2013/007755 décrit un procédé permettant de stériliser une émulsion dermo-cosmétique à un niveau de stérilité élevé, jusqu'à un F0 de 22, par un traitement thermique consistant en un chauffage direct de l'émulsion par infusion de vapeur.

Ce procédé donne satisfaction pour des formulations dont la viscosité est comprise entre 600 cP et 45 000 cP, c'est-à-dire pour des produits dermo-cosmétiques allant du lait au baume.

Toutefois, la mise en oeuvre de ce procédé nécessite que l'émulsion soit suffisamment fluide lors de son passage dans l'infuseur. Si l'émulsion n'est pas suffisamment fluide, celle-ci risque d'être « cassée », donc de perdre ses propriétés, lors du processus d'infusion, mais aussi, la productivité est réduite du fait des débits limités par la viscosité de l'émulsion. Selon ce document, l'amélioration de la fluidité de l'émulsion en vue de son traitement par infusion est obtenue en préchauffant ladite émulsion.

D'une manière générale, plus l'émulsion peut être préchauffée à une température élevée et plus elle est fluide. Mais, si la température de préchauffage est trop élevée il y a également un risque de casser l'émulsion.

Selon ce document, ce problème technique est résolu en déterminant une température de préchauffage, dite température limite de stabilité, par un essai de viscosité dynamique en température. La température, ou la plage de température, de préchauffage déterminées par cette méthode constituent un compromis entre une émulsion trop froide ou trop chaude lors de son injection dans l'infuseur, et permettent ainsi d'éviter de casser de manière irréversible l'émulsion lors de ce traitement.

La température, dite limite de stabilité selon ce document, constitue par conséquent une température ou une plage de température optimale de préchauffage pour faire subir au produit émulsionné considéré une opération de stérilisation par infusion.

Néanmoins, de nombreuses formulations dermo-cosmétiques chauffées à cette température optimale présentent encore une viscosité relativement élevée, de sorte que même si ce procédé de l'art antérieur reste pertinent, les débits de traitement atteignables sont limités, au moins en partie, du fait de la viscosité du produit à faire transiter dans les différents postes de l'installation.

**Résumé de l'invention**

L'invention vise à résoudre ces inconvénients et concerne à cette fin un procédé continu pour la stérilisation d'un produit dermo-cosmétique émulsionné, comprenant une base émulsive pour 90% à 99% du volume du produit composée d'au moins un composant aqueux et un composant gras, et des produits additionnels, dans lequel le débit est le même dans toute l'installation mettant en oeuvre les étapes iii) et iv), l'ensemble de la base émulsive et des produits additionnels constituant une formulation, lequel procédé comprenant les étapes consistant à :

i. déterminer un couple de données comportant une combinaison de composants de la formulation, dite formulation partielle, comprenant au moins la base émulsive, d'une viscosité η inférieure à 500cP à une température de préchauffage T1 comprise entre 80 et 90°C ;
ii. obtenir une pré-émulsion en mélangeant sous agitation en environnement non stérile les composants de la formulation partielle déterminée en i) ;
iii. préchauffer la pré-émulsion obtenue à l'étape ii) à la température T1 déterminée à l'étape i) ;
iv. stériliser par infusion la pré-émulsion préchauffée à l'étape iii) par un procédé par infusion de vapeur comprenant la pulvérisation en jet de la pré-émulsion dans une enceinte remplie de vapeur d'eau purifiée, portée à une température T2, suivie d'un poste de maintien à la température T2 et d'un poste de refroidissement rapide à une température T3, pour obtenir un abattement d'au moins 15Log du contenu microbien de la pré-émulsion (indice de stérilité F0 égal ou supérieur à 15) ;
v. stériliser séparément de la pré-émulsion les autres composants de la formulation ;
vi. mélanger sous agitation et en environnement stérile la pré-émulsion stérile obtenue à l'étape iv) avec les composants stériles, stérilisés à l'étape v) de sorte à obtenir le produit dermo-cosmétique émulsionné stérile, selon la formulation.

L'émulsion partielle chauffée à la température T1 conserve sa viscosité dynamique en fonction du taux de cisaillement dans un essai consistant à soumettre ladite émulsion partielle à des taux de cisaillement croissants puis décroissants compris entre 10 s⁻¹ et 150 s⁻¹.

Ainsi, le procédé objet de l'invention permet de bénéficier d'une forte productivité pour la mise en oeuvre de la stérilisation par infusion en n'appliquant celle-ci qu'à une partie de la formulation du produit final correspondant à une base émulsive, les autres composants étant ajoutés, stériles, individuellement ou en combinaison, après cette phase de stérilisation par infusion.

La pré-émulsion est une émulsion obtenue en émulsionnant au moins deux des composés de la formulation complète, en milieu non stérile, ce qui simplifie grandement cette opération la rendant plus productive.

Les autres composants de la formulation sont ajoutés au produit, sous environnement stérile, alors que la pré-émulsion, elle-même stérile, est déjà créée, ce qui requière beaucoup moins d'énergie, facilite la création de conditions stériles pour cette opération et augmente également la productivité. Les autres composants, ajoutés après la stérilisation de la pré-émulsion, sont stérilisés par toute méthode adaptée.

Le procédé selon l'invention peut en outre répondre aux caractéristiques de l'une quelconque des revendications 2 à 6.

L'invention peut avantageusement être mise en oeuvre selon les modes de réalisation et les variantes exposés ci-après, lesquels sont à considérer individuellement ou selon toute combinaison techniquement opérante.

Avantageusement la formulation partielle est sélectionnée de sorte à obtenir une pré-émulsion de viscosité $\eta$ inférieure à 300 cP à la température T1. Cette faible viscosité permet d'obtenir des débits de traitement très élevés dans un procédé de traitement continu.

Selon un mode de réalisation avantageux l'étape ii) est réalisée à une température égale à T1. Ce mode de réalisation permet de combiner en une même étape les étapes ii) et iii) du procédé en dirigeant directement la pré-émulsion obtenue lors de l'étape ii) vers le poste de stérilisation par infusion, sans passer par un poste de préchauffage spécifique.

La température T1 est comprise entre 80°C et 90°C. Cette plage de température est en effet favorable pour l'incorporation de certains composants dans la pré-émulsion et ce niveau de température relativement élevé permet en même temps d'obtenir une pré-émulsion fluide et de limiter l'amplitude du choc thermique subi par cette pré-émulsion lors de la phase de stérilisation.

L'étape i) du procédé objet de l'invention est réalisée par la mise en oeuvre d'essais de viscosité dynamique en température et par l'observation de l'évolution de la viscosité de la formulation partielle émulsionnée lorsqu'elle est soumise à des taux de cisaillement croissants puis décroissants compris entre 10 s$^{-1}$ et 150 s$^{-1}$ à la température T1. La réalisation de ces essais permet une sélection fine de la formulation partielle constituant la pré-émulsion ainsi que de la température optimale de préchauffage, correspondant à la température maximale de préchauffage à laquelle la pré-émulsion conserve ses caractéristiques de viscosité au cours de l'essais.

Avantageusement, l'étape ii) du procédé objet de l'invention consiste à obtenir une pré-émulsion grossière comprenant des tailles et une répartition des micelles non homogènes. Ainsi le temps de fabrication est encore réduit.

Selon un mode de réalisation particulier la formulation comprend un gélifiant sous la forme d'un copolymère acide et la pré-émulsion comprend ledit copolymère non neutralisé, l'étape vi) comprenant la neutralisation du copolymère. Ce mode de réalisation permet de bénéficier de tout l'effet viscosant et stabilisant du gélifiant dans le produit final, sans augmenter la viscosité de la pré-émulsion durant l'opération de stérilisation.

Une installation pour la mise en oeuvre du procédé objet de l'invention selon son mode de réalisation comprenant le passage direct de l'étape ii) à l'étape iv), cette installation n'étant pas objet de la présente invention, comprend :

    a. un premier poste de malaxage en température et en environnement non stérile pour obtenir une pré-émulsion grossière ;
    b. un poste de stérilisation par infusion ;

    c. un deuxième poste de malaxage en température et en environnement stérile à la sortie du poste de stérilisation et des moyens pour introduire dans ce deuxième poste de malaxage les composants stériles de la formulation non présents dans la pré-émulsion ;

dans lequel la pré-émulsion, obtenue dans le premier poste de malaxage à la température T1 est introduite directement dans le poste de stérilisation par infusion à la température T1 sans passer par un moyen de préchauffage intermédiaire.

Cette installation permet d'augmenter le débit de traitement et de réduire le temps de fabrication du produit dermo-cosmétique.

## Brève description des dessins

L'invention est exposée ci-après selon ses modes de réalisation préférés, nullement limitatifs, et en référence aux figures 1 à 7, dans lesquelles :

- la figure 1 est un exemple d'évolution en température de la viscosité d'une émulsion pour la détermination de la température limite de stabilité, figure 1A à une température inférieure à cette température, figure 1B à cette température et figure 1C pour une température supérieure à cette température limite de stabilité ;
- la figure 2 présente un synoptique de la mise en oeuvre d'un procédé selon un exemple de réalisation de l'invention mettant en oeuvre un premier exemple d'installation ;
- la figure 3 représente schématiquement un exemple d'installation pour la mise en oeuvre d'un des modes de réalisation du procédé objet de l'invention ;
- la figure 4 est un organigramme du procédé objet de l'invention selon ses différentes variantes ;
- la figure 5 représente des exemples de micrographies d'une émulsion, figure 5A une émulsion grossière et figure 5B une émulsion fine ;
- la figure 6 montre des exemples de répartition et d'homogénéité des micelles dans une émulsion fine et dans une émulsion grossière, telles qu'elles peuvent être obtenues à partir d'une analyse d'image de micrographies semblables à celles de la figure 5, la figure 6A représente un exemple de distribution de la dimension des micelles, la figure 6B montre un exemple de distributions des distances entre les micelles et leurs plus proches voisins ;
- et la figure 7 représente schématiquement le cycle thermique subi et l'évolution de la viscosité correspondante au cours de la fabrication d'un produit dermo-cosmétique selon un mode de réalisation du procédé objet de l'invention et au cours d'un procédé de l'art antérieur. L'échelle de temps n'est pas respectée, en particulier la phase de stérilisation est nettement dilatée en regard du reste du procédé.

## Description des modes de réalisation

L'invention s'applique à la fabrication un produit dermo-cosmétique sous la forme d'une émulsion. Un tel produit comprend au moins deux phases non miscibles, l'une des phases, dite phase interne ou dispersée, étant dispersée dans l'autre, dite phase externe ou continue.

Dans les produits concernés par l'invention, la phase interne est condensée ou liquide et la phase externe est liquide. Plus généralement l'une des phases interne ou externe est un produit aqueux et l'autre est un produit gras.

L'émulsion est obtenue en séparant et en brisant les gouttes de la phase interne de sorte à créer une dispersion homogène de celles-ci dans la phase externe.

Cette dispersion est obtenue par l'intermédiaire d'un effet mécanique, l'énergie mécanique ainsi introduite, par agitation ou injection, dans le produit, est stockée dans la tension superficielle des gouttes aux interphases. Le produit est ainsi homogène lorsque la taille des gouttes est fine est sensiblement la même dans toute l'émulsion.

Typiquement l'émulsion est fine lorsque la taille des micelles est inférieure à 3 $\mu$m ($3.10^{-3}$ mm), ce critère n'étant pas limitatif et dépendant de la nature de l'émulsion. Cette opération requière beaucoup d'énergie et reste complexe à réaliser dans un environnement stérile, de sorte qu'en termes de productivité il est plus avantageux de stériliser le produit obtenu après que celui-ci ait été émulsionné.

En dehors de la phase externe et de la phase interne qui constituent la base de l'émulsion, un produit dermo-cosmétique comprend d'autres composants, le plus souvent au moins une dizaine de composants.

Ces composants correspondent à des principes actifs spécifiques et à des composants améliorant les qualités organoleptiques du produit de sorte à lui conférer, par exemple, sa texture et la stabilité de celle-ci dans le temps.

Ces composés additionnels sont susceptibles de se dissoudre ou de se lier dans ou avec l'une des phases ou d'être en suspension dans la base émulsive.

Ces composants additionnels sont ainsi susceptibles de modifier les propriétés de l'émulsion, par exemple, en modifiant la tension superficielle aux interphases ou en se liant par adsorption ou par liaison covalente avec la phase dispersée créant éventuellement une réticulation entre ces phases, ou encore par effet de gélification.

Aussi, ces composants, au moins pour certains d'entre eux, modifient les propriétés physiques et notamment les propriétés de viscosité en fonction de la température. Ils peuvent ainsi stabiliser l'émulsion vis-à-vis de la température ou la rendre plus instable.

Le procédé de stérilisation par infusion d'une infusion dermo-cosmétique tel que décrit dans le document WO 2013/007755, requière que le produit soit porté à une température correspondant à un créneau précis pour que celle-ci soit à la fois suffisamment fluide mais suffisamment « robuste » pour résister à ce traitement sans que l'émulsion ne soit cassée.

Parmi les composants de la formulation, certains sont susceptibles, seuls ou en combinaison, de déplacer ce créneau de température optimale vers les hautes ou les basses températures ou encore de modifier la viscosité de l'émulsion dans un même créneau de température, en comparaison d'une formulation partielle ne comprenant pas ces composants.

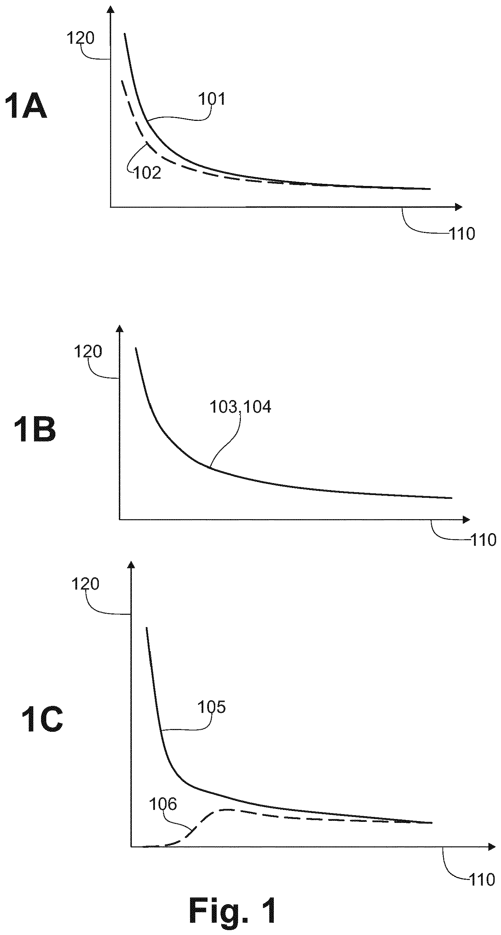

Figure 1, selon l'art antérieur décrit dans WO 2013/007755, la température optimale de préchauffage, en vue de sa stérilisation par infusion, d'une formulation émulsionnée comprenant, par exemple, les composants A, B, C et D, est déterminée à partir d'essais de viscosité dynamique consistant, pour une température donnée, à soumettre ladite formulation complète (A+B+C+D) à des taux de cisaillement (110), ou gradients de vitesses de cisaillement, croissants (101, 103, 105), pour obtenir une évolution de la viscosité $\eta$ (120) selon une courbe « aller », puis à des taux de cisaillement décroissants (102, 104, 106), pour obtenir une évolution de la viscosité selon une courbe « retour ».

Cette analyse est réalisée par exemple au moyen d'un rhéomètre, comprenant un mobile (cylindre ou cône) cisaillant l'émulsion entre la paroi dudit mobile et une paroi fixe. L'essai est par exemple réalisé en faisant varier le taux de cisaillement (110) entre 10 $s^{-1}$ et 150 $s^{-1}$.

Ce cycle de sollicitation permet d'appréhender qualitativement l'effet des sollicitations subies par le produit au cours d'un procédé de stérilisation thermique en continu tel que celui utilisé dans le procédé objet de l'invention, sur ses caractéristiques de viscosité et de stabilité de l'émulsion.

Figure 1A, lorsque la température à laquelle est réalisé l'essai est inférieure à la température optimale de préchauffage de la formulation émulsionnée, la courbe retour (102) enregistre des valeurs de viscosité plus faibles que la courbe aller (101) pour un même taux de cisaillement, car l'agitation du mobile tend à fluidifier l'émulsion.

Figure 1C, lorsque la température à laquelle est réalisé l'essai est supérieure à la température optimale de préchauffage de la formulation, les effets combinés du cisaillement et de la température à l'aller (105) détruisent l'émulsion, ce qui se traduit sur la courbe retour (106) par l'enregistrement d'une viscosité plus faible que sur la courbe aller.

Figure 1B, lorsque l'essai est pratiqué à une température ou dans une gamme de température correspondant à la température optimale de préchauffage, les courbes aller et retour sont quasiment superposées (aux incertitudes de mesure près), ce qui signifie que l'émulsion conserve globalement ses caractéristiques après avoir été sollicitée thermomécaniquement.

Ainsi, ces essais permettent, pour une formulation émulsive donnée, de déterminer une température ou une plage de température optimale de préchauffage en vue d'une stérilisation par infusion, ainsi que la viscosité de la formulation dans cette plage de température.

Les essais rhéologiques sont avantageusement complétés par des observations microscopiques de la structure de l'émulsion, notamment pour déterminer la taille et l'homogénéité des micelles pour une caractérisation plus précise de la température optimale de préchauffage.

C'est en portant ladite formulation à cette température optimale lors d'un préchauffage précédant la stérilisation par infusion, que celle-ci est la plus robuste vis-à-vis des sollicitations subies au cours de ce traitement et que les risques de casser de manière irréversible l'émulsion au cours du traitement sont minimisés, voir supprimés.

C'est ce qui est utilisé dans le procédé décrit dans le document WO 2013/007755. La solution technique est efficace, mais présente peu de flexibilité et limite les produits stérilisables selon ce procédé ainsi que les débits atteignables.

En effet, la température optimale de préchauffage ainsi déterminée pour la formulation complète émulsionnée du produit ne permet pas toujours d'atteindre un débit élevé dans un procédé continu du fait de la contre-pression engendrée par les pertes de charges dynamiques liées à la viscosité du produit.

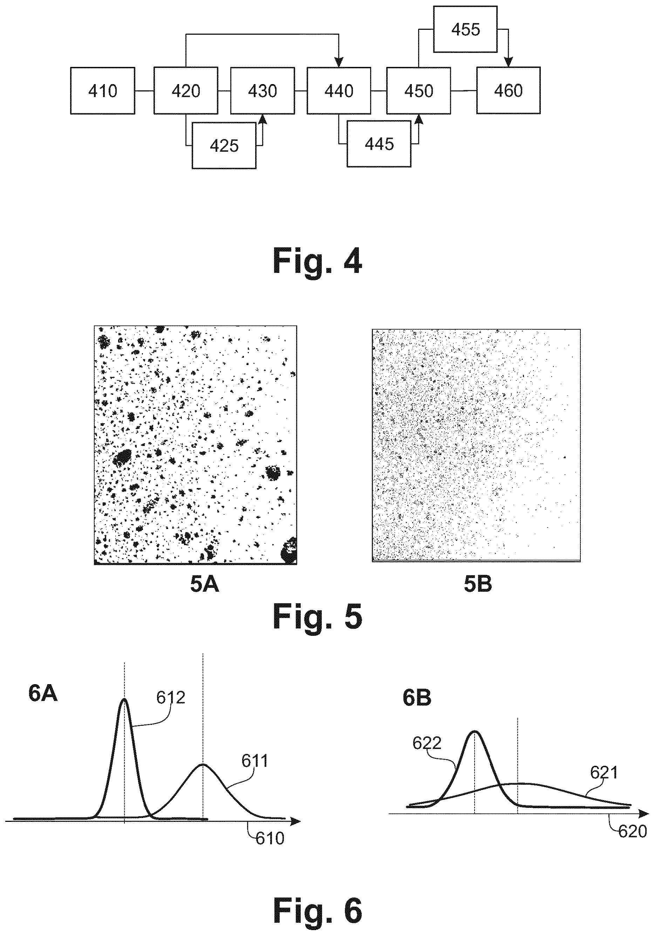

Figure 4, le procédé objet de l'invention résout ce problème en sélectionnant, au cours d'une étape de sélection (410), une formulation partielle comprenant une partie seulement des composants de la formulation complète pour obtenir une pré-émulsion que sera stérilisée par infusion selon un procédé continu.

En revenant à la figure 1, selon un exemple de mise en oeuvre du procédé objet de l'invention, cette étape de sélection est réalisée, selon le procédé objet de l'invention, en conduisant, non seulement pour une formulation complète (A+B+C+D) des essais de viscosité dynamique en température, mais aussi pour des formulations partielles, par exemple, (A+B), la formulation (A+B) constituant la base émulsive, (A+B+C) et (A+B+D).

Ces essais permettent alors de déterminer pour chaque formulation partielle, une température ou une plage de température optimales de préchauffage et une viscosité associée.

La température ou la plage de température optimales de préchauffage, sont celles où l'émulsion, ou pré-émulsion, consistant en la formulation partielle considérée (A+B, A+B+C, ou A+B+D) présente lors de l'essai un comportement correspondant à celui de la figure 1B explicitée ci-dessus, c'est-à-dire qu'elle ne perd pas ses propriétés après avoir subi les sollicitations thermomécaniques correspondantes, mais aussi pour laquelle la viscosité est la plus faible.

Ces essais mettent avantageusement en oeuvre la technique des plans d'expériences, par exemple de type TAGUCHI. Ainsi pour un produit comprenant N composants en plus de la base émulsive la formulation partielle est déterminées à partir de N essais de ce type, et même moins, dans la mesure où l'influence de certains composants sur la viscosité est connue.

La baisse de viscosité obtenue en utilisant une formulation partielle (en comparaison de la formulation complète) est liée soit à l'influence d'un ou de plusieurs composants de la formulation complète sur cette viscosité ou à la capacité de cette formulation partielle de supporter une température de préchauffage plus élevée et par suite de pouvoir être amenée à un niveau de fluidité plus élevé.

Dans l'absolu, le couple formulation partielle et température optimale de préchauffage présentant la viscosité la plus faible est celui offrant potentiellement la meilleure productivité.

Toutefois, la sélection finale de la formulation partielle n'est pas obligatoirement basée sur cette sélection optimale et prend en considération d'autres facteurs de compromis comme la facilité d'ajouter les composants à la pré-émulsion avant ou après sa stérilisation par infusion, en tenant compte du fait qu'une formulation partielle offrant en tant que pré-émulsion une viscosité inférieure ou égale à 500 cP, idéalement inférieure ou égale à 300 cP à la température optimale de préchauffage, répond à l'objectif de productivité.

De même, il n'est pas nécessaire de tester toutes les formulations partielles possibles, l'expérience permettant de fixer une formulation partielle ou un jeu de formulations partielles candidates présentant a priori les meilleurs compromis, ceci notamment en fonction de l'effet connu de certains composants tels que des gélifiants ou des épaississants sur les propriétés rhéologiques du produit.

Ainsi, selon le procédé objet de l'invention, la formulation partielle retenue comme pré-émulsion, intégrant la base émulsive, est celle qui présente une viscosité adaptée à une température de préchauffage optimale T1, laquelle température T1 est déterminée au besoin par des essais de viscosité dynamique en température.

En revenant à la figure 4, la sélection (410) de la formulation partielle ayant été réalisée, les composants correspondants sont mélangés et émulsionnés en environnement non stérile de sorte à obtenir une pré-émulsion au cours d'une étape (420) de mélange.

Cette étape (420) comprend le mélange sous une agitation suffisante d'au moins un composant aqueux et d'un composant gras constituant une base émulsive.

Pour les produits dermo-cosmétiques visés par l'invention, cette base émulsive correspond à 90 % voire 99 % du volume du produit final.

Cette opération de mélange et d'émulsion est généralement réalisée « à chaud », c'est-à-dire à une température comprise entre 40°C et 85°C, selon la nature des composants.

Ainsi, selon des variantes de mise en oeuvre du procédé objet de l'invention, la pré-émulsion obtenue à l'issue de l'étape de mélange et d'émulsion (420) est refroidie et stockée (425) avant de subir une étape (430) de préchauffage jusqu'à la température optimale de préchauffage, ou, la pré-émulsion obtenue est directement dirigée, chaude, vers l'étape de préchauffage (430) en économisant du temps et de l'énergie pour ce préchauffage, ou encore, lorsque la température d'obtention de la pré-émulsion à l'étape de mélange (420) se trouve dans la plage de température optimale de préchauffage, ladite pré-émulsion est directement dirigée vers l'étape de stérilisation par infusion (440), économisant ainsi toute l'étape de préchauffage (430).

Ainsi, selon un exemple non limitatif, la formulation comprend un facteur de consistance liposoluble, apte à augmenter la viscosité du produit final par un phénomène d'épaississement physique.

Ce facteur de consistance est par exemple une substance solide à température ambiante, ayant un point de fusion supérieur à la température ambiante, par exemple supérieur à 50 °C ou 60 °C. Un tel composant doit être incorporé dans la phase grasse constituant la pré-émulsion à une température supérieure à son point de fusion, température à laquelle il ne modifie pas la viscosité de la pré-émulsion.

Ainsi selon cet exemple non limitatif, la formulation partielle de la pré-émulsion comprend ledit facteur de consistance liposoluble dans sa phase grasse, et la pré-émulsion est obtenue au cours de la phase de mélange (420) à une température de 80 °C ou de 85 °C puis est dirigée directement vers l'étape (440) de stérilisation par infusion sans préchauffage complémentaire. Un exemple de facteur de consistance est un corps gras cireux dépourvu de propriété émulsionnante, par exemple un hydrocarbure saturé à chaîne carbonée en C18-C30.

Selon un autre exemple non limitatif de mise en oeuvre, la formulation du produit comprend un gélifiant tel qu'un copolymère acide. Ce type de gélifiant, contenu dans la phase aqueuse, participe à la stabilité du produit final, éventuellement en coopération avec le facteur de consistance.

Selon un premier mode de réalisation la formulation partielle retenue pour la constitution de la pré-émulsion ne comprend pas ledit gélifiant et celui-ci est introduit lors de l'étape (450) de finalisation.

Selon un autre mode de mise en oeuvre, le gélifiant est un copolymère acide et son effet de gélification n'est obtenu qu'en neutralisant celui-ci par l'ajout d'une base organique ou minérale. Cette neutralisation permet le déroulement des chaînes du copolymère et son action sur la consistance du produit. Ainsi, selon cet exemple, la formulation partielle correspondant à la pré-émulsion comprend le gélifiant à l'état non neutralisé.

Ainsi selon cet exemple de réalisation, pour l'obtention de la pré-émulsion, un tel gélifiant est intégré, dispersé puis hydraté dans la phase aqueuse à une température de l'ordre de 40°C.

La phase grasse de la pré-émulsion étant préparée à une température comprise entre 80°C et 90°C, la phase aqueuse comprenant le gélifiant à l'état non neutralisé ou partiellement neutralisé est portée également à une température comprise entre 80°C et 90°C avant d'être mélangée et émulsionnée avec la phase grasse.

Ainsi l'étape de mélange (420) est réalisée à une température comprise entre 80°C et 90°C et la pré-émulsion ainsi obtenue est dirigée directement vers l'étape (440) de stérilisation par infusion. La neutralisation du gélifiant intervient après l'étape de stérilisation (440), au cours de l'étape de finalisation (450), par l'ajout à la pré-émulsion des composants alcalins adaptés pour réaliser la neutralisation du gélifiant.

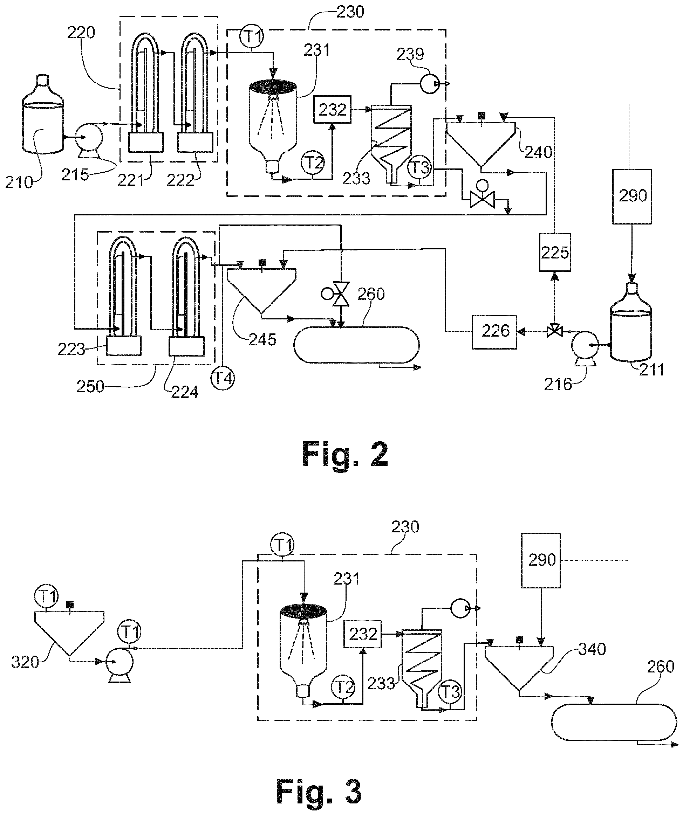

L'étape (440) de stérilisation par infusion comprend elle-même trois étapes décrites plus en détails en référence aux modes de réalisation exposés figures 2 et 3 :

- une étape de chauffage direct par infusion de vapeur de la pré-émulsion à la température de stérilisation ;
- une étape de chambrage assurant un maintien de la pré-émulsion à la température de stérilisation pendant un temps assurant le degré de stérilité voulu de la pré-émulsion ;
- une étape de refroidissement rapide, ou flash, de la pré-émulsion jusqu'à une température proche de la température de préchauffage à laquelle est portée la pré-émulsion juste avant l'étape de stérilisation.

Selon un mode de réalisation avantageux, la pré-émulsion ainsi stérilisée et se trouvant à une température comprise entre T1 et T1-5°C à l'issue du refroidissement flash, est dirigée vers un malaxeur stérile à cette même température dans lequel elle est mélangée, selon une étape de finalisation (450) du produit, aux autres composants de la formulation afin d'obtenir le produit dermo-cosmétique.

Ainsi, selon un des exemples cités précédemment, la pré-émulsion se trouve à une température comprise entre 80°C et 90°C à l'issue de l'étape de stérilisation et est mélangée avec les autres composants de la formulation à cette même température lors de l'étape de finalisation (450).

À titre d'exemple, la pré-émulsion comprenant un gélifiant à l'état non neutralisé, l'étape de finalisation (450) comprend l'ajout sous agitation à ladite pré-émulsion et sous environnement stérile des composants permettant de neutraliser le gélifiant. Lesdits composants ont été stérilisés préalablement à leur incorporation dans le malaxeur stérile, cette stérilisation étant réalisée par toute méthode adaptée connue de l'art antérieur.

Alternativement, la pré-émulsion se trouvant à une température comprise entre T1 et T1-5°C est d'abord refroidie, au cours d'une étape de refroidissement (445) jusqu'à une température T4 inférieure à T1, et l'étape de finalisation (450) est réalisée à cette température T4.

Selon, une variante de réalisation, l'étape de finalisation comprend une étape de finalisation supplémentaire (455) consistant à mélanger d'autres composants stériles, sous agitation et sous environnement stérile à une température inférieure à celle de la première étape (450) de finalisation du produit.

Ainsi, selon un exemple de mise en oeuvre, une première étape (450) de finalisation du produit est réalisée en additionnant à la pré-émulsion stérilisée, et à une température de 80°C, certains composants de la formulation, puis la température du malaxeur est descendue à 40°C et d'autres composants de la formulation sont ajoutés à cette température réduite, par exemple les additifs permettant de neutraliser le gélifiant.

Le produit finalisé est ensuite stocké (460) en cuve stérile en vue de son conditionnement.

Le procédé objet de l'invention est un procédé de traitement continu d'un lot de produit.

Dans un procédé de traitement continu, le débit de traitement est fixé par les moyens de pompage répartis dans l'installation et qui amènent le produit d'un poste de traitement à un autre.

Le débit atteignable est limité, entre autres, par la pression de pompage. Cette pression est notamment le résultat des pertes de charges dynamiques subies par le produit transitant dans les différents conduits et moyens de l'installation.

Ainsi, le débit maximum possible est limité notamment par la pression maximale qui peut être utilisée lors du pompage, cette pression maximale étant limitée par la technologie des pompes utilisées mais aussi par l'effet de cette pression sur la qualité et les propriétés du produit. D'une manière générale moins le produit est visqueux et plus les débits atteignables sont élevés.

Figure 2, selon un exemple de réalisation, la formulation partielle du produit stérilisée par infusion ayant été déterminée (étape 410 figure 4), celle-ci est contenue sous la forme d'une pré-émulsion, c'est-à-dire comprenant au moins deux composants de la formulation complète et constituant une base émulsive, dans un réservoir (210).

Préalablement à la réalisation d'un nouveau lot l'ensemble de l'installation est nettoyé et stérilisé.

Des moyens de pompage (215) permettent d'amener le produit vers une station de préchauffage (220), et dans tous les postes de l'installation.

Selon des exemples de réalisation, l'installation comprend plusieurs moyens de pompage répartis dans celle-ci mais le débit reste bien entendu le même dans toute l'installation. Le débit atteint dépend notamment de la viscosité de la pré-émulsion.

Ladite pré-émulsion est portée dans la station de préchauffage (220) à une température (T1) comprise dans la plage de température optimale de préchauffage de la formulation partielle telle que déterminée par exemple au moyen d'essais rhéologiques en température tels que décrits *supra.*

La station de préchauffage (220) comprend des moyens de chauffage indirects, tels qu'un ou plusieurs échangeurs (221, 222) à parois raclées, en série ou en parallèle.

La disposition en série permet de réaliser un chauffage par palier, la disposition en parallèle permet de passer un plus gros débit.

Un tel échangeur thermique comprend un stator et un rotor pourvu de pâles. Le stator comprend une double paroi chauffée à la température désirée par une circulation fluide, notamment de la vapeur, de sorte que la paroi interne de l'échangeur (221, 222) se trouve à une température de consigne, la paroi du dernier des échangeurs étant fixée à la température T1.

Ces échangeurs assurent une montée progressive et homogène en température de la pré-émulsion, avec une vitesse de préchauffage comprise entre 0,1°C.s⁻¹ et 1°C.s⁻¹ , par exemple.

Après le préchauffage, la pré-émulsion est amenée vers la station de stérilisation (230). La stérilisation est réalisée selon un procédé dit par infusion.

La station de stérilisation (230) comprend un poste de chauffage par infusion (231), un poste de maintien en température (232) et un poste de refroidissement rapide (233) dit « flash ». Le chauffage par infusion est réalisé en pulvérisant en jet la pré-émulsion dans une enceinte remplie de vapeur d'eau purifiée, portée à la température désirée.

La surface d'échange du produit avec la vapeur étant très élevée, le chauffage de la pré-émulsion à la température de stérilisation T2 est quasiment instantané dans tout le volume injecté dans la chambre de stérilisation. Au cours de cet échauffement, la pré-émulsion absorbe de l'eau correspondant à la condensation de la quantité de vapeur lui ayant transmis sa chaleur.

Le poste de maintien en température (132) permet de maintenir la pré-émulsion ainsi chauffé à la température atteinte lors du chauffage par infusion, afin d'obtenir le niveau de stérilité visé. Ce poste de maintien, ou de chambrage, consiste en un conduit de longueur adaptée en fonction du débit et situé entre le poste de chauffage par infusion (231) et le poste de refroidissement (233). La longueur du conduit définit ainsi le temps de maintien.

À l'issue de ce maintien, la pré-émulsion, à la température de stérilisation T2, entre dans le poste de refroidissement (233).

Dans ce poste de refroidissement, la pré-émulsion, chauffée à la température T2, est mise en communication avec une chambre mise sous vide par des moyens appropriés (239).

Le produit aspiré dans cette chambre subit une détente brusque accompagnée d'une libération violente de vapeur. La chaleur latente de vaporisation prélève de l'énergie thermique au produit et ainsi le refroidit, jusqu'à une température T3.

L'eau est récupérée sous forme de vapeur dans le haut du refroidisseur, alors que la pré-émulsion, débarrassée de l'eau absorbée lors du chauffage par infusion, est collecté et récupérée en bas du refroidisseur.

Le cycle thermodynamique est réglé de sorte que l'eau absorbée lors du chauffage par infusion du cycle de stérilisation est récupérée sous forme de vapeur lors du cycle de refroidissement flash. Ainsi, les températures T1 et T3 sont nécessairement proches, et T3 est comprise entre T1 et T1-5°C.

Parallèlement à ces opérations, selon un exemple de réalisation, les autres composants devant être ajoutés à la pé-émulsion pour constituer le produit dermo-cosmétique final, passent par un poste de stérilisation (290) dont la méthode de stérilisation est adaptée à la nature du composant et sont stockés stériles dans un réservoir (211).

Selon des variantes de réalisation, lesdits composants sont mélangés dans un même réservoir, ou le dispositif comprend plusieurs réservoirs correspondant aux différents composants.

Selon un mode de réalisation, la pré-émulsion stérilisée par infusion et se trouvant à une température T3, est mélangée sous agitation avec les composants complémentaires dans un mélangeur stérile à une température proche de la température T3.

Dans ce cas, lesdits composants, amenés par une pompe (216), sont par exemple préchauffés si nécessaire, à cette même température ou une température proche de celle-ci, dans un poste de préchauffage (225) indirect.

Selon un autre mode de réalisation, la pré-émulsion est d'abord refroidie par un poste de refroidissement (250) jusqu'à une température T4 inférieure à la température T3.

Le poste de refroidissement comprend par exemple un ou plusieurs échangeurs à surface raclée (223, 224) en série ou en parallèle pour à la fois passer le débit désiré et abaisser progressivement la température de la pré-émulsion à la température T4.

Les composants complémentaires sont mélangés à la pré-émulsion à la température T4 dans un mélangeur (245) stérile de technologie adaptée, après avoir été éventuellement préchauffés à la température T4 ou une température proche de celle-ci dans un poste de chauffage (226) indirect.

Le produit correspondant à la formulation complète est stocké dans un réservoir stérile (260) pour être transféré vers un poste de conditionnement en environnement stérile.

Le dispositif selon ce premier mode de réalisation est adapté à la mise en oeuvre du procédé décrit dans le document WO 2013/007755 où la formulation complète émulsionnée est traitée en stérilisation par infusion, avec des débits atteignant, selon cet art antérieur 2m$^3$/h.

Toutefois, la mise en oeuvre du procédé objet de l'invention dans cette même installation permet d'atteindre des débits de traitement plus élevés, notamment avec des formulations visqueuses ou comprenant des gélifiants, en passant par une pré-émulsion correspondant à une formulation partielle, stérilisée par infusion tel que décrit ci-avant.

Avantageusement la vapeur générée au niveau du poste (230) de chauffage par infusion permet de stériliser l'ensemble de l'installation.

Selon les variantes de réalisation, les conditions de traitement : T1, T2, T3, T4, et de débit sont adaptées au produit traité, mais plus avantageusement les conditions sont fixées pour couvrir une large gamme de produits et le choix des produits transitant par les différents postes de l'installation : pré-émulsion et composants stériles ajoutés après stérilisation par infusion de la pré-émulsion, sont sélectionnés par la méthode explicitée *supra* pour s'adapter à ces conditions et à l'obtention de débits de traitement plus élevés.

La température de préchauffage de la pré-émulsion est fixée entre 80°C et 90°C, cette température permet d'obtenir une pré-émulsion particulièrement fluide.

La température de stérilisation T2 est fixée entre 141°C et 145°C pour un temps de maintien dans le poste de chambrage (232) compris entre respectivement 10 secondes et 6 secondes de sorte à atteindre un niveau de stérilité F0 supérieur à 15. La température T3 est également comprise entre 80°C et 90°C et la température T4 est comprise entre 30°C et 40 °C.

Avantageusement le procédé objet de l'invention permet de stériliser la base émulsive de la formulation, éventuellement associée à quelques composants et qui constitue l'essentiel du volume de la formulation, à un débit très élevé, et de stériliser les autres composants, présents en nettement moins grande quantité selon des procédés moins productifs en termes de débit, le tout selon un procédé continu, conservant le débit de la stérilisation par infusion imposé à la pré-émulsion, ceci en ajustant les températures et le choix de la formulation partielle de la pré-émulsion, pour amener celle-ci à une viscosité adaptée au débit visé.

Figure 3, selon un autre mode de réalisation, les avantages du procédé objet de l'invention permettent de simplifier l'installation de stérilisation en continue et d'atteindre des débits de traitement encore plus élevés.

En effet, la sélection d'une pré-émulsion de formulation partielle selon le procédé objet de l'invention entraîne généralement une température optimale de préchauffage élevée, et dans de nombreux cas, ladite pré-émulsion de formulation partielle supporte également des conditions de préchauffage ou de refroidissement plus sévères de sorte que les postes de préchauffage ou de refroidissement progressifs (220 et 250 figure 2) mettant en oeuvre des échangeurs à surface à raclée (221, 222, 223, 224 figure 2) ne sont pas nécessaires. La suppression des échangeurs à surface raclée qui contiennent un volume important de produit, simplifie l'installation, limite les pertes de produit traité et facilite le nettoyage et la stérilisation de l'installation.

Ainsi, selon ce mode de mise en oeuvre, l'installation de la figure 2 est simplifiée et comprend un poste de mélange et d'émulsion (320) de la pré-émulsion comprenant les composants de la formulation partielle sélectionnée, dans lequel la température finale de réalisation de la pré-émulsion est égale à la température optimale de préchauffage T1 de ladite pré-émulsion.

À cette fin le poste de mélange (320) comprend des moyens de chauffage pour en contrôler la température. Cette opération est réalisée en environnement non stérile.

Selon des variantes de réalisation les composants de la pré-émulsion sont introduits dans le mélangeur à des températures croissantes et des niveaux d'agitation différents.

Par exemple, une partie des composants est introduite et mélangée à une température de 40°C sous agitation forte et les autres composants sont introduits à une température comprise entre 80°C et 90°C, sous agitation plus faible, de sorte qu'à l'issue de cette opération, la pré-émulsion obtenue est à une température T1, comprise entre 80°C et 90°C, et dans la plage de température optimale de préchauffage de ladite pré-émulsion.

Ainsi, en sortant du poste de mélange (320) à la température T1, la pré-émulsion est dirigée vers le poste (230) de stérilisation par infusion.

La stérilisation, par infusion est réalisée selon les mêmes étapes et conditions que décrites en regard de la figure 2.

À l'issue de la stérilisation, la pré-émulsion, à la température T3 proche de la température T1 est dirigée vers un poste de malaxage (340) stérile, lui-même à la température T3 et comprenant des moyens pour en contrôler la température.

Les autres composants de la formulation sont alors ajoutés à la pré-émulsion à la température T3 après avoir eux-mêmes subi une étape (290) de stérilisation par tout procédé adapté.

À titre d'exemple non limitatif, les composants additionnels sont stérilisés par une ultrafiltration pour les composants liquides. Un tel procédé d'ultrafiltration à 0,22 $\mu$m (0,22.10$^{-3}$ mm) produit également un abattement du nombre de germes dans le produit filtré égale ou supérieur à 15 Log, ce faible diamètre de porosité empêchant le passage des bactéries.

Les composants non liquides sont stérilisés par toute méthode adaptée.

Comme pour la réalisation du mélange initial correspondant à la pré-émulsion, le mélange final est, selon des variantes de réalisation, réalisé selon une séquence comprenant plusieurs températures décroissantes et plusieurs niveaux d'agitation, ainsi, par exemple, une partie des composants est introduite à la température T3 sous agitation forte et une autre partie des composants est introduite à une température inférieure à T3, par exemple 40°C, sous agitation plus faible.

Quelle que soit l'installation utilisée pour la mise en oeuvre du procédé objet de l'invention, celui-ci présente des avantages supplémentaires participant à l'augmentation de la productivité.

Ainsi, il est constaté, d'une part, que le procédé de stérilisation par infusion de la pré-émulsion permet de réduire la taille des micelles de la phase grasse dans la phase aqueuse de l'émulsion et d'obtenir une répartition plus homogène de la phase grasse dans la phase aqueuse en comparaison de l'état de la pré-émulsion avant la stérilisation.

De plus, il est constaté que l'énergie de cisaillement / malaxage lors de la phase de finalisation, où la pré-émulsion stérilisée est mélangée avec les autres composants de la formulation, est inférieure à l'énergie de malaxage qui serait nécessaire pour obtenir le produit correspondant à la formulation complète sans passer par la phase de pré-émulsion stérilisée par infusion.

Ainsi, la pré-émulsion peut être obtenue de manière grossière en mettant en oeuvre une première opération de malaxage (420 figure 4), rapide, tout en obtenant *in fine* un produit homogène avec une fine taille de micelles, typiquement comprise entre 2 $\mu$m et 3 $\mu$m à l'issue de la phase de finalisation (450 figure 4), le temps total de malaxage dépensé aux deux postes de malaxage du procédé objet de l'invention restant inférieur au temps de malaxage nécessaire à l'obtention du produit stérilisé selon le procédé de stérilisation de l'art antérieur.

Ainsi, non seulement le procédé objet de l'invention permet d'augmenter considérablement les débits de traitement en passant d'un débit de 2m$^3$/h selon l'art antérieur, à un débit de 4m$^3$/h pour un même produit sur une installation mettant en oeuvre le même poste (230 figures 2 et 3) de stérilisation par infusion, mais également de réduire le temps de fabrication d'un lot de 24 heures à 14 heures.

Figure 5, la qualité d'une l'émulsion en termes de taille des micelles et d'homogénéité est par exemple déterminée de manière qualitative par des observations micrographiques de l'émulsion, et de manière quantitative par des analyses d'images desdites observations micrographiques.

La figure 5A montre un exemple de micrographie d'une émulsion grossière, et la figure 5B un exemple d'une micrographie d'une émulsion fine. Sur ces images la phase grasse apparaît comme des tâches sombres et la phase aqueuse apparaît en fond blanc.

Les deux micrographies, figure 5A et figure 5B sont à la même échelle.

Figure 5A qualitativement, dans l'émulsion grossière, la phase grasse se répartie selon des micelles de dimension variable et comportant des micelles de diamètre important, de quelques dizaines de micromètres, et réparties de manière non homogène sur une surface d'analyse donnée.

Figure 5B, dans l'émulsion fine les micelles sont de taille homogène, de petite dimension et réparties de manière homogène sur une surface d'analyse donnée.

Figure 6, ces paramètres sont quantifiables par une analyse d'image en appréciant, par mesure et comptage, figure 6A la distribution statistique de la taille (610) des micelles sur une surface d'analyse et figure 6B en appréciant par mesure et comptage, la distribution statistique de la distance (620) entre micelles les plus proches sur cette même surface d'analyse. Les distributions (612, 622) correspondant à l'émulsion fine ont une variance plus faible que les distributions (611, 621) correspondant à l'émulsion grossière. Dans la pratique, les distributions obtenues ne sont pas nécessairement gaussiennes.

L'obtention d'une émulsion fine comprenant des micelles de petite dimension homogènes en taille et bien réparties, participe à l'efficacité du produit dermo-cosmétique, conférant audit produit une texture particulièrement agréable au contact de la peau, moins collante, plus facile et plus rapide à étaler. Le produit est également plus stable vis-à-vis des phénomènes de relargage, déphasage, précipitation, coalescence, floculation, crémage, etc. et conserve ainsi ses propriétés organoleptiques dans le temps. La finesse des micelles permet lors d'une application topique, une pénétration plus facile de celles-ci entre les cornéocytes, et un comblement plus rapide et efficace des lamelles lipidiques protectrices de la peau. Le comblement lipidique cornéocytaire en est d'autant plus rémanent.

Le procédé objet de l'invention permet ainsi d'obtenir ces avantages sur le produit final en partant d'une pré-émulsion grossière, et ainsi de fabriquer le produit dermo-cosmétique plus rapidement, mais avec une qualité accrue en regard du procédé de l'art antérieur.

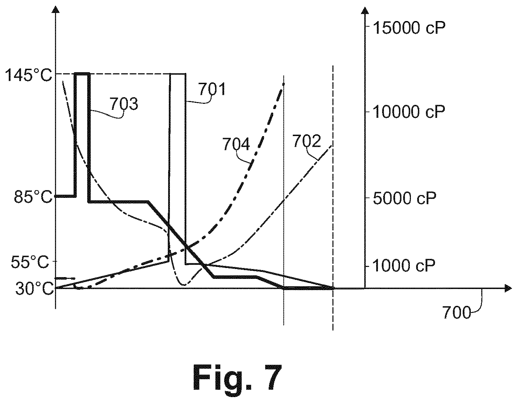

La figure 7 compare, en fonction du temps (700), le cycle thermique (701) subit par un produit visqueux, tel qu'un baume, transitant dans une installation de stérilisation par infusion selon l'art antérieur, ainsi que l'évolution de sa viscosité (702), en traits fins, au cycle thermique (703) subit par le même produit transitant dans une installation mettant en oeuvre un mode de réalisation du procédé objet de l'invention correspondant à l'installation représentée figure 3 ainsi que l'évolution de sa viscosité (704), en traits forts.

Selon le procédé de l'art antérieur, le produit transitant dans l'installation, c'est-à-dire la formulation complète du produit cosmétique émulsionnée, subit des variations importantes de viscosité.

Particulièrement, l'émulsion est initialement très visqueuse et est progressivement fluidifiée par un chauffage progressif, puis, suite à la stérilisation par infusion, la viscosité du produit augmente à nouveau au cours de la phase de refroidissement progressif.

La viscosité du produit en fin de cycle est constatée plus faible que celle du produit initialement émulsionné.

Du fait de ces variations importantes de viscosité, l'obtention d'un débit continu dans l'installation nécessite la présence de zones tampons dans la progression du produit. Ces zones tampons sont en pratique constituées par les postes de chauffage et de refroidissement progressif dans les échangeurs thermiques à surface raclée. Aussi, dans une installation mettant en oeuvre le procédé de l'art antérieur, le débit de traitement atteignable est également limité, indépendamment des capacités des moyens de pompage, par le nombre d'échangeurs thermiques présents pour le préchauffage et le refroidissement progressif.

Une installation donnée comprend un nombre fixe de tels échangeurs thermiques, de sorte que plus le produit traité est initialement visqueux, plus la variation de viscosité au cours du cycle est importante et plus le débit pratiquement atteignable est faible.

Avec le procédé objet de l'invention, selon le mode de réalisation considéré, la pré-émulsion pénètre dans l'installation de stérilisation avec une faible viscosité, inférieure ou égale à 300 cP, et déjà à la température T1, qui plus est supérieure à la température de préchauffage selon l'art antérieur.

La viscosité du produit en transit ne croît qu'à la fin du cycle lorsque les composants complémentaires sont ajoutés, pour atteindre *in fine* une viscosité du produit complet supérieure à celle obtenue par le procédé de l'art antérieur.

Rien ne limite le débit si ce n'est la capacité des moyens de pompage et la capacité d'obtenir le mélange final au cours de l'étape de finalisation en un temps suffisamment court, compatible avec le débit visé.

Or, d'une part, ce temps de finalisation est inférieur au temps de refroidissement progressif du produit selon le procédé de l'art antérieur, et la pré-émulsion qui constitue la très grande partie du volume du produit a par ailleurs été affinée au cours de l'étape de stérilisation par infusion, de sorte que le temps de malaxage nécessaire au cours de l'étape de finalisation est très inférieur à celui qui est nécessaire pour l'obtention du produit correspondant à la formulation complète selon le procédé de l'art antérieur.

En ajoutant à ces effets que la pré-émulsion obtenue selon le procédé objet de l'invention est grossière et par conséquent est rapidement créée, les gains de productivité du procédé objet de l'invention sont en pratique multiples et se répartissent tout le long du cycle de fabrication.

La description ci-avant et les exemples de réalisation, montrent que le procédé objet de l'invention atteint le but visé et permet d'augmenter la productivité de la fabrication d'un produit dermo-cosmétique stérile tout en restant compatible avec les installations de stérilisation de l'art antérieur, quel que soit son mode de mise en oeuvre. Le procédé objet de l'invention offre plus de flexibilité dans les conditions de traitement et permet de traiter dans des conditions de productivité élevée des produits dermo-cosmétiques, notamment très visqueux, difficiles à traiter selon le procédé de l'art antérieur.

**Revendications**

1. Procédé continu pour la stérilisation d'un produit dermo-cosmétique émulsionné, comprenant une base émulsive pour 90% à 99% du volume du produit composée d'au moins un composant aqueux et un composant gras et des produits additionnels, dans lequel le débit est le même dans toute l'installation mettant en oeuvre les étapes iii) et iv), l'ensemble de la base émulsive et des produits additionnels constituant une formulation, lequel procédé comprend les étapes consistant à :

i. déterminer (410) un couple de données comportant une combinaison de composants de la formulation, dite formulation partielle et comprenant la base émulsive, d'une viscosité η inférieure à 500cP à une température de préchauffage T1 comprise entre 80°C et 90°C ;

ii. obtenir (420) une pré-émulsion en mélangeant sous agitation en environnement non stérile les composants de la formulation partielle déterminée en i) ;

iii. préchauffer (430) la pré-émulsion obtenue à l'étape ii) à la température T1 déterminée à l'étape i) ;

iv. stériliser (440) la pré-émulsion préchauffée à l'étape iii) par un procédé par infusion de vapeur comprenant la pulvérisation en jet de la pré-émulsion dans une enceinte remplie de vapeur d'eau purifiée, portée à une température T2, suivie d'un poste (232) de maintien à la température T2 et d'un poste de refroidissement rapide (233) à une température T3, pour obtenir un abattement d'au moins 15Log du contenu microbien de la pré-émulsion ;

v. stériliser séparément de la pré-émulsion les autres composants de la formulation ;

vi. mélanger (450) sous agitation et en environnement stérile la pré-émulsion stérile obtenue à l'étape iv) avec les composants stériles, stérilisés à l'étape v) de sorte à obtenir le produit dermo-cosmétique émulsionné stérile, selon la formulation ;

**caractérisé en ce que** l'émulsion partielle chauffée à la température T1, conserve sa viscosité dynamique en fonction du taux de cisaillement dans un essai consistant à soumettre ladite émulsion partielle à des taux de cisaillement croissants puis décroissants compris entre 10 s$^{-1}$ et 150 s$^{-1}$.

2. Procédé selon la revendication 1, dans lequel la formulation partielle est sélectionnée de sorte à obtenir une pré-émulsion de viscosité η inférieure ou égale à 300 cP à la température T1.

3. Procédé selon la revendication 1, dans lequel l'étape ii) est réalisée à une température égale à T1, et l'étape iv) est réalisée sans étape iii) préalable.

4. Procédé selon la revendication 1, dans lequel T2 est comprise entre 141°C et 145°C, T3 est comprise entre 80°C et 90°C, le temps de maintien à la température T2 au poste de maintien (232) étant compris entre 10 secondes et 6 secondes de sorte à obtenir un abattement d'au moins 15Log du contenu microbien à la température T2 et dans lequel l'étape vi) de mélange (450) est réalisée à une température T4 comprise entre 30°C et 40 °C.

5. Procédé selon la revendication 1, dans lequel l'étape v) comprend une stérilisation par ultrafiltration à 0,22 μm desdits autres composants liquides.

6. Procédé selon la revendication 1, dans lequel la formulation comprend un gélifiant sous la forme d'un copolymère acide et la pré-émulsion comprend ledit copolymère non neutralisé, l'étape vi) comprenant la neutralisation du copolymère.

## Patentansprüche

1. Kontinuierliches Verfahren zur Sterilisation eines emulgierten dermo-kosmetischen Produkts, umfassend eine emulgierende Basis für 90 % bis 99 % des Produktvolumens, die aus mindestens einer wässrigen Komponente und einer Fettkomponenten zusammengesetzt ist, und zusätzliche Produkte, wobei die Durchflussrate in der gesamten Anlage, in der die Schritte iii) und iv) durchgeführt werden, gleich ist, wobei die gesamte emulgierende Basis und die zusätzlichen Produkte eine Formulierung darstellen, wobei das Verfahren die Schritte umfasst, die aus Folgendem bestehen:

i. Bestimmen (410) eines Datenpaars, das eine Kombination von Komponenten der Formulierung, die Teilformulierung genannt wird und die emulgierende Basis mit einer Viskosität η von kleiner als 500 cP bei einer Vorwärmtemperatur T1 zwischen 80 °C und 90 °C umfasst;

ii. Erhalten (420) einer Voremulsion durch Mischen der Komponenten der in i) bestimmten Teilformulierung unter Rühren in einer nicht sterilen Umgebung;

iii. Vorwärmen (430) der in Schritt ii) erhaltenen Voremulsion auf die in Schritt i) bestimmte Temperatur T1;

iv. Sterilisieren (440) der in Schritt iii) vorgewärmten Voremulsion durch ein Dampfinfusionsverfahren, das das Sprühstrahlen der Voremulsion in eine Kammer umfasst, die mit gereinigtem Wasserdampf gefüllt ist, der auf eine Temperatur T2 gebracht wird, gefolgt von einer Station (232) zum Halten der Temperatur T2 und einer Station zum schnellen Abkühlen (233) auf eine Temperatur T3, um eine Absenkung des mikrobiellen Gehalts der Voremulsion um mindestens 15Log zu erreichen;

v. getrenntes Sterilisieren der Voremulsion von den anderen Komponenten der Formulierung;

vi. Mischen (450) unter Rühren und in einer sterilen Umgebung der in Schritt iv) erhaltenen sterilen Voremulsion mit den in Schritt v) sterilisierten sterilen Komponenten, um das sterile emulgierte dermo-kosmetische Produkt gemäß der Formulierung zu erhalten;

**dadurch gekennzeichnet, dass** die auf die Temperatur T1 erwärmte Teilemulsion ihre dynamische Viskosität als Funktion der Schergeschwindigkeit in einem Test beibehält, der darin besteht, die Teilemulsion zunehmenden und dann abnehmenden Schergeschwindigkeiten zwischen 10 s$^{-1}$ und 150 s$^{-1}$ auszusetzen.

2. Verfahren nach Anspruch 1, wobei die Teilformulierung derart ausgewählt wird, dass eine Voremulsion mit einer Viskosität $\eta$ von kleiner gleich 300 cP bei der Temperatur T1 erhalten wird.

3. Verfahren nach Anspruch 1, wobei der Schritt ii) bei einer Temperatur gleich T1 durchgeführt wird und der Schritt iv) ohne den vorherigen Schritt iii) durchgeführt wird.

4. Verfahren nach Anspruch 1, wobei T2 zwischen 141 °C und 145 °C beträgt, T3 zwischen 80 °C und 90 °C beträgt, die Zeit zum Halten der Temperatur T2 bei der Haltestation (232) zwischen 10 Sekunden und 6 Sekunden beträgt, um eine Absenkung des mikrobiellen Gehalts um mindestens 15Log bei der Temperatur T2 zu erreichen, und wobei der Schritt vi) des Mischens (450) bei einer Temperatur T4 zwischen 30 °C und 40 °C durchgeführt wird.

5. Verfahren nach Anspruch 1, wobei der Schritt v) eine Sterilisation durch Ultrafiltrierung bei 0,22 um der anderen flüssigen Komponenten umfasst.

6. Verfahren nach Anspruch 1, wobei die Formulierung ein Geliermittel in Form eines sauren Copolymers umfasst und die Voremulsion das nicht neutralisierte Copolymer umfasst, wobei Schritt vi) die Neutralisation des Copolymers umfasst.

**Claims**

1. Continuous method for the sterilisation of an emulsified dermo-cosmetic product, comprising an emulsive base for 90% to 99% by volume of the product composed of at least an aqueous component and a fatty component and additional products, wherein the flow rate is the same throughout the plant implementing steps iii) and iv), the whole formed by the emulsive base and the additional products constituting a formulation, which method comprises the steps of:

i. determining (410) a data pair including a combination of components of the formulation, so-called partial formulation and comprising the emulsive base, with a viscosity $\eta$ lower than 500 cP at a preheating temperature T1 comprised between 80°C and 90°C;

ii. obtaining (420) a pre-emulsion by mixing under stirring in a non-sterile environment the components of the partial formulation determined in i);

iii. pre-heating (430) the pre-emulsion obtained in step ii) at the temperature T1 determined in step i);

iv. sterilising (440) the pre-emulsion pre-heated in step iii) through a vapour-infusion process comprising jet-spraying the pre-emulsion in a chamber filled with purified water vapour, brought at a temperature T2, followed by a station (232) for holding at the temperature T2 and a station (233) for rapid cooling down to a temperature T3, to obtain a reduction by at least 15 Log of the microbial content of the pre-emulsion;

v. sterilising the other components of the formulation separately from the pre-emulsion;

vi. mixing (450), under stirring and in a sterile environment, the sterile pre-emulsion obtained in step iv) with the sterile components, sterilised in step v) so as to obtain the sterile emulsified dermo-cosmetic product, according to the formulation;

**characterised in that** the partial emulsion heated at the temperature T1, retains its dynamic viscosity as a function of the shear rate in a test consisting in subjecting said partial emulsion to increasing and then decreasing shear rates comprised between 10 s$^{-1}$ and 150 s$^{-1}$.

2. Method according to claim 1, wherein the partial formulation is selected so as to obtain a pre-emulsion with a viscosity $\eta$ lower than or equal to 300 cP at the temperature T1.

3. Method according to claim 1, wherein step ii) is carried out at a temperature equal to T1, and step iv) is carried out without prior step iii).

4. Method according to claim 1, wherein T2 is comprised between 141°C and 145°C, T3 is comprised between 80°C and 90°C, the hold time at the temperature T2 at the holding station (232) being comprised between 10 seconds and 6 seconds so as to obtain a reduction by at least 15 Log of the microbial content at the temperature T2 and wherein the mixing step vi) (450) is carried out at a temperature T4 comprised between 30°C and 40°C.

5. Method according to claim 1, wherein step v) comprises a sterilization by 0.22 um ultra-filtration of said other liquid components.

6. Method according to claim 1, wherein the formulation comprises a gelling agent in the form of an acid copolymer and the pre-emulsion comprises said non-neutralised copolymer, step vi) comprising neutral-ising the copolymer.

Fig. 1

# Fig. 2

# Fig. 3

**Fig. 4**

5A

5B

**Fig. 5**

**Fig. 6**

**Fig. 7**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 1588697 A1
- WO 9932156 A2
- WO 2013007755 A